# EUROPÄISCHE PATENTANMELDUNG

(11) **EP 4 757 115 A1**
(43) Veröffentlichungstag der Anmeldung: **10.06.2026**
(21) Anmeldenummer: 25209676.3
(22) Anmeldetag: 20.10.2025
(51) Int. Cl.: H02J 7/50, G16H 20/40, G16H 40/63, H02J 7/82

(54) **SYSTEM UND VERFAHREN ZUR ZENTRALEN ÜBERWACHUNG UND STEUERUNG VON DEN JEWEILIGEN BATTERIELADEVORGANG MEHRERER BATTERIEBETRIEBENER MEDIZINISCHER GERÄTE**

(30) Priorität: 06.12.2024 DE 102024136531
(71) Anmelder: Karl Storz SE & Co. KG, 78532 Tuttlingen (DE)
(72) Erfinder: Wenzler, Sebastian, 78532 Tuttlingen (DE); Hinding, Thomas, 78532 Tuttlingen (DE)

(57) **Zusammenfassung**

Die Erfindung schafft ein System sowie ein computerimplementiertes Verfahren zur zentralen Überwachung und Steuerung eines jeweiligen Batterieladevorgangs mehrerer batteriebetriebener medizinischer Geräte (G1, G2, G3). Das System (100) umfasst:
eine Eingangsschnittstelle (10), welche dazu eingerichtet ist, Batterieparameterdaten (D0) der medizinischen Geräte (G1, G2, G3) zu erfassen;
eine Recheneinrichtung (20), welche dazu eingerichtet ist, basierend auf den Batterieparameterdaten (D0) einen Batterieladezustand der medizinischen Geräte (G1, G2, G3) zu ermitteln;
eine Ausgabeschnittstelle (30), welche dazu eingerichtet ist, ein Ausgabesignal (A1) zu erzeugen, welches mindestens einem Batterieladezustand der medizinischen Geräte (G1, G2, G3) entspricht; und
eine Steuerungseinrichtung (40), welche dazu eingerichtet ist, basierend auf dem erzeugten Ausgabesignal (A1) einen jeweiligen Batterieladevorgang der medizinischen Geräte (G1, G2, G3) zu steuern.

## Beschreibung

### Technisches Gebiet der Erfindung

Die vorliegende Erfindung betrifft ein System zur zentralen Überwachung und Steuerung eines jeweiligen Batterieladevorgangs mehrerer batteriebetriebener medizinischer Geräte, Instrumenten oder Zubehör, insbesondere für Geräte, Instrumente oder Zubehör, die für chirurgische Eingriffe eingesetzt werden, dessen Batterien selbst im Gerät oder in Batterieladestationen geladen werden. Die Erfindung betrifft ferner ein computerimplementiertes Verfahren zur zentralen Überwachung und Steuerung eines jeweiligen Batterieladevorgangs mehrerer batteriebetriebener medizinischer Geräte, Instrumenten oder Zubehör.

### Hintergrund der Erfindung

Mit modernen Technologien im Gesundheitssektor nimmt die Anzahl von batteriebetriebenen Geräten, die z.B. in Operationssälen einer medizinischen Einrichtung (Krankenhäuser, Kliniken) eingesetzt werden, ständig zu. Je nach Operation können sehr unterschiedliche Instrumente zum Einsatz kommen, die zum erforderlichen Zeitpunkt einsatzbereit sein müssen.

Es ist daher wichtig, insbesondere bei Operationen, dass alle zu verwendenden Geräte bedarfsgerecht, z.B. gemäß einer OP-Planung, einsatzbereit zur Verfügung stehen.

Informationen über den Batterieladezustand eines Gerätes werden üblicherweise durch eine visuelle Inspektion des Gerätes gewonnen, die in der Regel durch das Personal einer medizinischen Einrichtung durchgeführt wird. Wird der Batterieladezustand eines Gerätes als zu niedrig bewertet, müssen die Batterien entweder am Gerät oder an einer Ladestation aufgeladen werden. Dieses Vorgehen muss für jedes Gerät vom Krankenhauspersonal durchgeführt werden, da der Ladezustand bisher nur direkt am Gerät oder an einer Ladestation angezeigt wird.

Mit der zunehmenden Anzahl von batteriebetriebenen Geräten ist dieses Vorgehen nicht nur zeitaufwändig, sondern muss auch auf einer Schätzung beruhen. Der Leser des Batterieladezustands muss selbst beurteilen, ob der Batterieladezustand eines Gerätes für eine geplante Anwendung ausreichend ist. Dies hängt wiederum vom Batteriezustand (Alterungszustand) des Gerätes ab, d.h. vom Gesundheitszustand der Batterie, der schwer einzuschätzen ist. Weiterhin, wenn eine Batterie geladen werden muss, muss sichergestellt werden, dass die Ladestationen nicht belegt sind. Eine solche Überwachung und Steuerungsplanung in der Größenordnung eines Krankenhauses ist vom Menschen allein schwer zu verwalten.

Vor diesem Hintergrund ist es daher notwendig, eine verbesserte Ermittlung des Batterieladezustands der unterschiedlichen batteriebetriebenen Geräte, die in den verschiedenen medizinischen Operationen und Prozeduren verwendet werden, bereitzustellen.

### Zusammenfassung der Erfindung

Es ist daher eine Aufgabe der vorliegenden Erfindung, ein System und ein Verfahren bereitzustellen, um den Batterieladezustand eines oder mehrerer Geräte zentral zu überwachen und den Batterieladevorgang jedes einzelnen Gerätes, in Abhängigkeit der geplanten Anwendung des Gerätes und unter Einbeziehung möglicher Komplikationen und Verzögerungen zu steuern.

Diese Aufgabe wird durch die Gegenstände der unabhängigen Patentansprüche der vorliegenden Erfindung gelöst.

Gemäß einem ersten Aspekt der Erfindung wird demnach ein System zur zentralen Überwachung und Steuerung eines jeweiligen Batterieladevorgangs mehrerer batteriebetriebener medizinischer Geräte bereitgestellt. Das System umfasst mindestens eine Eingangsschnittstelle, welche dazu eingerichtet ist, Batterieparameterdaten der mehreren medizinischen Geräte zu erfassen; eine Recheneinrichtung, welche dazu eingerichtet ist, basierend auf den erfassten Batterieparameterdaten einen jeweiligen Batterieladezustand der mehreren medizinischen Geräte zu ermitteln; eine Ausgabeschnittstelle, welche dazu eingerichtet ist, ein erstes Ausgabesignal zu erzeugen, welches mindestens einem der ermittelten Batterieladezustand der mehreren medizinischen Geräte entspricht; und eine Steuerungseinrichtung, welche dazu eingerichtet ist, basierend auf dem erzeugten ersten Ausgabesignal einen jeweiligen Batterieladevorgang der mehreren medizinischen Geräte zu steuern.

Eine grundlegende Idee der vorliegenden Erfindung besteht darin, dass die Verwaltung (darunter eine Überwachung und eine Steuerung) des Batterieladezustands jedes batteriebetriebenen Geräts einer medizinischen Einrichtung zentral erfolgen kann. Dadurch kann der Batterieladezustand einer Mehrzahl von Geräten, Instrumenten und Zubehör kontinuierlich überwacht (oder: getrackt) werden und frühzeitig ein notwendiger Batteriewechsel bzw. Gerätewechsel oder auch Akkuwechsel signalisiert werden.

Somit wird die Verfügbarkeit der Geräte, der Instrumente und des Zubehörs ermittelt. Daher wird das Risiko einer leeren Batterie oder eines nicht einsatzbereiten Gerätes minimiert. Die Erfindung stellt daher ein System bereit, das die Anwendungen von Instrumenten und Geräten in der komplexen Struktur einer medizinischen Einrichtung verwalten kann und sorgt ferner dafür, dass die Ladung- und Entladungszyklen der Instrumente und Geräte optimal durchgeführt werden können. Insbesondere das Laden eines Gerätes kann in Abhängigkeit der geplanten Verwendung angepasst gesteuert werden. Somit kann das Laden insbesondere unter Berücksichtigung (oder: mit dem Ziel) einer verbesserten Lebensdauer der Batterie optimiert werden.

Der Begriff "batteriebetriebene medizinische Geräte" wird hier weit gefasst und umfasst auch Instrumente und Zubehör, die mit Batterien betrieben sind, und die in einer medizinischen Einrichtung für eine geplante Prozedur verwendet werden können. "Batteriebetriebene medizinische Geräte" umfassen beispielsweise unter anderem Fußschalter, HF/Ultraschall-Versiegelungsinstrumente, oszillierende Sägen, Perfusionspumpen, Elektrokardiogrammgeräte, Stirnlampen, Endoskope, Kameraeinheiten oder Bohrmaschinen. Das Laden der Batterie eines batteriebetriebenen Geräts, oder das Überwachen des Ladevorgangs einer Batterie, wird der Einfachheit halber hierin auch kurz als "Laden des batteriebetriebenen Geräts" oder auch nur als "Laden des Geräts" bezeichnet, bzw. als "Überwachen des Ladevorgangs des Geräts".

Unter einer Überwachung ist insbesondere ein kontinuierliches Erfassen von Batterieparameterdaten, die zur Ermittlung eines Batterieladezustands beitragen, zu verstehen. Die Eingangsschnittstelle, die Recheneinrichtung und die Ausgabeschnittstelle sind unter anderen dazu eingerichtet, eine solche Überwachungsfunktion durchzuführen.

Bei Batterieparameter handelt es sich um physikalische Größe (wie etwa Temperatur oder Spannung), die gemessen werden kann und eine Information über die Batterie liefert. Ein Batterieparameter könnte aber auch eine Information, die vom Hersteller stammt, wie etwa die Chemie der Batterie oder die Kapazität der Batterie, enthalten. Auch das Alter der Batterie oder Information über die historischen Ladungs- und Entladungszyklen der Batterie sind als Batterieparameter anzusehen. Batterieparameterdaten sind Daten über mindestens einen Batterieparameter.

Batterieparameterdaten können hier Bilddaten (die aus einer externen Bildgebungseinrichtung übermittelt werden) sowie Textdaten oder Daten aus verschiedenen Messungen, beispielsweise thermischen und/oder elektrischen Messungen, umfassen. Es kann sich um Rohdaten oder schon vorbearbeiteten Daten handeln.

Obwohl hier, im Vorstehenden und im Folgenden einige Funktionen als von "Einrichtungen", "Schnittstellen" oder "Modulen" ausgeführt beschrieben werden, versteht es sich, dass dies nicht unbedingt bedeutet, dass solche Einrichtungen, Schnittstellen, oder Module als voneinander getrennte Einheiten bereitgestellt werden. In Fällen, in denen eine oder mehrere Einrichtungen, Schnittstellen, oder Module ganz oder teilweise als Software bereitgestellt werden, können die Einrichtungen, Schnittstellen, oder Module durch Programmcodeabschnitte oder Programmcodeschnipsel implementiert werden, die sich voneinander unterscheiden, aber auch miteinander verwoben sein können.

In ähnlicher Weise können in dem Fall, in dem ein oder mehrere Einrichtungen, Schnittstellen, oder Module als Hardware bereitgestellt werden, die Funktionen einer oder mehrerer Einrichtungen, Schnittstellen, oder Module von ein und derselben Hardwarekomponente bereitgestellt werden, oder die Funktionen einer Einrichtung, einer Schnittstelle, oder eines Moduls, oder die Funktionen mehrerer Einrichtungen, Schnittstellen, oder Module können auf mehrere Hardwarekomponenten verteilt sein, die nicht notwendigerweise den Einrichtungen, Schnittstellen, oder Modulen eins zu eins entsprechen müssen. Daher ist jede Vorrichtung, jedes System, jedes Verfahren usw., das alle Merkmale und Funktionen aufweist, die einer bestimmten Einrichtung, einer Schnittstelle, und/oder einem bestimmen Modul zugeschrieben werden, so zu verstehen, dass sie bzw. es die Einrichtung, die Schnittstelle, und/oder das Modul ausbildet, umfasst, oder implementiert.

Insbesondere besteht die Möglichkeit, dass alle Schnittstelle, Einrichtungen und Module durch Programmcode implementiert werden, der von der Recheneinrichtung ausgeführt wird.

Die Recheneinrichtung kann als jede Vorrichtung oder jedes Mittel zum Rechnen realisiert werden, insbesondere zum Ausführen einer Software, einer App oder eines Algorithmus. Beispielsweise kann die Recheneinrichtung mindestens einen Prozessor, wie etwa mindestens einen Zentralprozessor, CPU und/oder mindestens einen Grafikprozessor, GPU, und/oder mindestens ein feldprogrammierbares Gate-Array, FPGA, und/oder mindestens einen anwendungsspezifischen integrierten Schaltkreis, ASIC, und/oder eine beliebige Kombination der Vorstehenden umfassen. Die Recheneinrichtung kann ferner einen Arbeitsspeicher aufweisen, der mit dem mindestens einen Prozessor operativ verbunden ist, und/oder einen nicht-flüchtigen Speicher, der mit dem mindestens einen Prozessor und/oder dem Arbeitsspeicher operativ verbunden ist. Die Recheneinrichtung kann teilweise und/oder vollständig in einer lokalen Vorrichtung und/oder teilweise und/oder vollständig in einem entfernten System, wie z.B. durch eine Cloud-Computing-Plattform, implementiert sein.

Die Eingangsschnittstelle kann dazu eingerichtet sein, die Batterieparameterdaten direkt von mindestens einer Messungseinheit, einer Bildgebungseinrichtung, oder durch eine Benutzerschnittstelle zu erhalten, insbesondere in Echtzeit, oder alternativ auch von einer Datenbank. Die Eingangsschnittstelle kann auch mindestens eine Bildgebungseinrichtung oder eine Messungseinheit umfassen, sodass das Erhalten der Batterieparameterdaten auch ein Erfassen der Batterieparameterdaten durch die Bildgebungseinrichtung und die Messungseinheit umfassen kann. Die Verarbeitung dieser Daten kann jedoch stets mit derselben Recheneinrichtung erfolgen.

Die Ausgabeschnittstelle kann eine Benutzerschnittstelle umfassen oder an einer Benutzerschnittstelle oder an einen zentralen Monitor verbunden sein. Somit kann das erste Ausgabesignal als eine graphische Darstellung des ermittelten Batterieladezustands der Geräte für einen Nutzer zur Verfügung stehen.

Weitere Vorteile der Erfindung werden im Folgenden anhand der Gegenstände der Unteransprüche und insbesondere anhand der Beschreibung der Figuren erläutert.

Gemäß einigen bevorzugten Ausführungsformen, Varianten oder Verfeinerungen von Ausführungsformen umfasst die Eingangsschnittstelle Sensoren, die dazu ausgelegt sind, elektrische und/oder thermische Batterieparameterdaten zu erfassen. Bei diesen Ausführungsformen kann die Eingangsschnittstelle die Batterieparameterdaten erhalten aber auch erfassen. Die Sensoren können die Entwicklung der Temperatur und der Spannung und/oder Strom der Batterie mit der Zeit messen. Die Sensoren können an Geräte angebracht werden, um das Entladen des Gerätes zu überwachen, und/oder an Ladestationen implementiert werden, um das Laden der verschiedenen Batterien zu überwachen. Diese Sensoren können beispielsweise durch Bluetooth, WLAN, ZigBee, NFC, Wibree oder WiMAX im Radiofrequenzbereich sowie IrDA, optischen Richtfunk (FSO) und/oder LiFi im infraroten bzw. optischen Frequenzbereich mit einer zentralen Empfangseinheit der Eingangsschnittstelle signalverbunden werden, um die gemessenen Batterieparameterdaten in Echtzeit zu übertragen. Die Daten der Sensoren können auch an einen Nutzer ausgegeben werden, z.B. als Teil des ersten Ausgabesignals der Ausgabeschnittstelle. Weiterhin kann diese Information mit einer Benutzerschnittstelle visualisiert werden.

Gemäß einigen bevorzugten Ausführungsformen, Varianten oder Verfeinerungen von Ausführungsformen ist die Steuerungseinrichtung mit mehreren Batterieladestationen verbindbar, um den jeweiligen Batterieladevorgang der mehreren medizinischen Geräte zu steuern. Wenn die Geräte keinen Akku besitzen müssen die Batterien entfernt werden und in Ladestationen geladen werden. Die Steuerungseinrichtung kann mit einer Anzahl von Batterieladestationen vernetzt werden, um die passende Batterieladestation für das Laden einer Batterie zu finden. Die Steuerungseinrichtung kann die Batterieladestation orchestrieren, um das Laden der zu ladenden Geräten in Abhängigkeit von der Ladezeit und die geplanten Anwendungszeit eines Gerätes optimal zu verwalten. Die Steuerungseinrichtung kann auch eine Zellbalancing-Funktion aufweisen, wonach alle die Zellen einer Batterie gleichmäßig geladen und entladen werden. Dies sorgt dafür, dass die Lebensdauer der Batterie optimiert wird.

Gemäß einigen bevorzugten Ausführungsformen, Varianten oder Verfeinerungen von Ausführungsformen umfasst das System der Erfindung ferner eine Energieverwaltungseinrichtung, welche dazu eingerichtet ist, einen Energieverbrauch der mehreren medizinischen Geräte zu optimieren. Die Energieverwaltungseinrichtung kann mit der Steuerungseinrichtung verbunden sein und daher eine automatische Abschaltung eines Gerätes zu veranlassen oder das Gerät in Standby zu setzen bei nicht Nutzung, um einen Energieverbrauch der Batterie zu optimieren. Die Energieverwaltungseinrichtung kann ein fehlerhaftes oder nicht optimales Laden und/oder Entladen einer Batterie erkennen und entsprechende Anweisungen an die Steuerungseinrichtung übermitteln. Die Energieverwaltungseinrichtung kann mit der Ausgabeschnittstelle auch verbunden sein, damit Vorwarnungen an einen Nutzer übermittelt werden könnten. In manchen Ausführungsformen kann die Energieverwaltungseinrichtung in der Recheneinrichtung implementiert werden.

Gemäß einigen bevorzugten Ausführungsformen, Varianten oder Verfeinerungen von Ausführungsformen umfasst die Recheneinrichtung ein künstliche-Intelligenz-Modul, welches eine künstliche-Intelligenz-Entität, KIE, implementiert, die dafür trainiert und eingerichtet ist, eine Lade- und/oder Entladestrategie der mehreren medizinischen Geräte zu erstellen. Diese Strategien können unter Berücksichtigung eines Modells der Batterie oder einer Simulation der Batterie erstellt werden. Die KIE kann auch Funktionen wie die Zellbalancing-Funktion der Steuerungseinrichtung unterstützen, oder die Verwaltung der Batterieladestationen (z.B. basierend auf Simulationen) optimieren. Die KIE kann beispielsweise den Entladezeitpunkt einer Batterie vorhersagen und diese mit einer vorhersagten Belegung der Batterieladestationen an dieser Zeit zu vergleichen, sodass ein Laden der Batterie in dem Netz der Batterieladestationen gewährleistet werden kann.

Unter einer KIE sind insbesondere Maschinenlernen-Modelle oder andere algorithmische Architekturen zu verstehen, die insbesondere unter Deep Learning eingestuft werden können. Bei der Künstliche-Intelligenz-Entität kann es sich beispielsweise um eine Support-Vector-Maschine, einen k-Means-Algorithmus, oder ein künstliches neuronales Netz handeln, beispielsweise ein Convolutional Neural Network (CNN).

Gemäß einigen bevorzugten Ausführungsformen, Varianten oder Verfeinerungen von Ausführungsformen basiert die Lade- und/oder Entladestrategie der mehreren medizinischen Geräte zumindest auf Informationen bezüglich einer OP-Planung. Die Verwaltung des Lade- und Entladevorgangs eines Gerätes ist besonders wichtig, um eine Anpassung an die OP-Planung einer medizinischen Einrichtung zu ermöglichen. In diesem Zusammenhang kann die OP-Planung an die Eingangsschnittstelle übermittelt werden. Die Instrumente, Zubehör und Geräte für die verschiedenen Operationen und Prozeduren können in der OP-Planung detailliert werden oder aus einer Datenbank abgerufen werden. Die OP-Planung kann auch Informationen über die Mindest-Batterieladezustand der Instrumente, Zubehör und Geräte für die verschiedenen Operationen darstellen, oder benutzerspezifische Einstellungen. Die Recheneinrichtung kann basierend auf der Angangszeit und Dauer einer Operation eine Lade- und Entladestrategie für die erforderlichen Geräte ermitteln. Die Recheneinrichtung kann ferner den voraussichtlichen Verbrauch der mehreren medizinischen Geräte ermitteln und einen Ladungsplan erzeugen, der von der Steuerungseinrichtung implementiert werden kann. Der Ladungsplan kann von einem Benutzer durch eine Benutzerschnittstelle visualisiert werden. Der Ladungsplan kann auch an einer Benutzerschnittstelle übertragen werden.

Gemäß einigen bevorzugten Ausführungsformen, Varianten oder Verfeinerungen von Ausführungsformen ist die KIE ferner dafür trainiert und eingerichtet, Abweichungen der OP-Planung zu erkennen und darauf basierend einen Batterieladezustand der mehreren medizinischen Geräte am Ende der OP entsprechend der OP-Planung vorherzusagen. Während einer Operation können insbesondere Komplikationen zu einer Verlängerung der Operation führen. Die KIE kann aus den Bilddaten eines Endoskops oder einer Raumkamera bzw. aus den Gerätedaten und den erkannten Phasen einer OP Abweichungen von der OP-Planung erkennen. Darauf basierend kann die KIE beurteilen, ob der jeweilige prognostizierte Batterieladezustand der verschiedenen in einer OP eingesetzten medizinischen Geräte bis zum Ende der OP ausreicht bzw. ob ein jeweils vordefinierter Mindestbatterieladezustand der medizinischen Geräte bis zum Ende der OP erreicht (und/oder beispielsweise unterschritten) wird.

Gemäß einigen bevorzugten Ausführungsformen, Varianten oder Verfeinerungen von Ausführungsformen umfasst das System der Erfindung ferner eine Benutzerschnittstelle, die dazu eingerichtet ist, Eingaben von einem Nutzer zu empfangen und Informationen über die Überwachung und Steuerung der medizinischen Geräte an eine Anzeigeeinrichtung zu übermitteln. Die Benutzerschnittstelle kann vorzugsweise eine graphische Schnittstelle umfassen. Eine solche Benutzerschnittstelle kann beispielsweise als graphische Benutzerschnittstelle durch eine Anzeigeeinrichtung des Systems implementiert sein, insbesondere durch einen Bildschirm oder einen Touchscreen. Die Benutzerschnittstelle kann die Kommunikation zwischen dem System der Erfindung und einem Nutzer erleichtern. Beispielsweise kann die Benutzerschnittstelle mit der Eingangsschnittstelle verbunden sein, um Informationen über die OP-Planungen oder Benutzerpräferenzen im Rahmen einer Prozedur zu erhalten. Die Benutzerstelle kann ebenfalls Ausgabesignalen der Ausgabeschnittstelle anzeigen, wie zum Beispiel den Batterieladezustand eines Gerätes, die ermittelte Ladeplanung oder die geplante Verwendung eines Gerätes gemäß einer OP-Planung.

Gemäß einigen bevorzugten Ausführungsformen, Varianten oder Verfeinerungen von Ausführungsformen umfasst die Recheneinrichtung eine Meldungseinrichtung, welche dazu eingerichtet ist, eine Anzahl der mehreren medizinischen Geräte auf der Grundlage mindestens einer OP-Planung auszuwählen und für jedes der ausgewählten medizinischen Geräte einen entsprechenden OP-Saal zuzuweisen. Durch die Meldungseinrichtung können somit die medizinischen Geräte je nach Operationsplanung automatisch aussortiert werden. Die Meldungseinrichtung daher gewährleistet, dass die für eine Operation benötigten medizinischen Geräte eigentlich identifiziert und einer spezifischen Operation zugeordnet werden.

Die Meldungseinrichtung kann auch dazu eingerichtet sein, Bildgebungsdaten oder Texteingabe von einer Benutzerschnittstelle zu erhalten. Die Meldungseinrichtung kann mit der graphischen Schnittstelle einer Benutzerschnittstelle verbunden oder gekoppelt sein, z.B. mit einem Tablet, sodass durch Scannen eines Barcodes oder QR-Codes, Instrumente, Zubehör oder Geräte automatisch identifiziert werden können.

Alternativ kann die Meldungseinrichtung eine Bilderkennungseinheit umfassen, mittels welcher die Meldungseinrichtung die vom Nutzer gesendeten Bilder automatisch erkennen kann. Die graphische Schnittstelle kann in machen Ausführungsformen eine App enthalten, die so konfiguriert ist, eine OP-Planung und die gewünschten Geräte an das System der Erfindung zu übermitteln und eine Darstellung des vom System ermittelten Batterieladezustands der Geräte, ihrer Verfügbarkeit und der Ladeplanung gemäß der OP-Planung zu erzeugen.

Gemäß einigen bevorzugten Ausführungsformen, Varianten oder Verfeinerungen von Ausführungsformen gibt die Meldungseinrichtung ein Warnsignal aus (d.h. ist dazu eingerichtet, ein Warnsignal auszugeben), wenn sich ein gruppiertes medizinisches Gerät nicht im vorgesehenen OP-Saal befindet. Die Meldungseinrichtung kann nicht nur ein spezifisches Gerät einer OP-Planung zuordnen, sondern auch die Position des Gerätes tracken. Das Warnsignal kann in einer Benutzerschnittstelle angezeigt werden. Es ist auch denkbar, dass ein akustisches Warnsignal ausgelöst wird, wenn das Gerät ohne vorherige Abmeldung aus dem zugehörigen OP-Raum entfernt wird. Damit wird gewährleistet, dass die bei einer Operation zu verwendenden Geräten nicht verwechselt oder vermisst werden.

Gemäß einigen bevorzugten Ausführungsformen, Varianten oder Verfeinerungen von Ausführungsformen ist die Steuerungseinrichtung ferner dazu eingerichtet, eine Notabschaltung eines Batterieladevorgangs durchzuführen, falls die zugehörigen Batterieparameterdaten außerhalb von bestimmten Werten liegen. Dadurch kann kritischen Situationen, wie etwa einer Überhitzung oder einem Kurzschluss bei einem Laden vorgebeugt werden, um Schäden an den Geräten zu verhindern.

Gemäß einigen bevorzugten Ausführungsformen, Varianten oder Verfeinerungen von Ausführungsformen ist die Ausgabeschnittstelle ferner dazu eingerichtet, ein zweites Ausgabesignal zu erzeugen, welches einen (notwendigen) Batteriewechsel und/oder einen Gerätewechsel signalisiert. Wenn die Recheneinrichtung feststellt, dass der Batterieladezustand eines Gerätes unter einem bestimmten Sollwert liegt, kann ein Ausgabesignal erzeugt werden, um einen Nutzer zu informieren, dass einen Wechsel erforderlich ist. Das zweite Ausgabesignal kann auch einen Akkuwechsel signalisieren. Der Sollwert für ein medizinisches Gerät kann von der Prozedur abhängig sein. Beispielsweise kann der Sollwert für eine kurze Operation niedriger sein als der Sollwert für eine lange Operation.

Gemäß einigen bevorzugten Ausführungsformen, Varianten oder Verfeinerungen von Ausführungsformen ist die Steuerungseinrichtung ferner dazu eingerichtet, auf Basis des zweiten Ausgabesignals, welches einen Batteriewechsel und/oder einen Gerätewechsel signalisiert, ein autonomes Transportsystem zu steuern. Ein oder mehrere autonome Transportfahrzeuge können im Krankenhaus dafür eingesetzt werden, die zu ladenden batteriebetriebenen Geräte einzusammeln und diese Geräte zu einer Ladestation bringen, wo sie aufgeladen werden (können). Die Transportfahrzeuge können auch die aufgeladenen Geräte gemäß einer oder mehrerer OP-Planungen an die entsprechenden Operationssäle eines Krankenhauses verteilen.

Gemäß einem zweiten Aspekt stellt die vorliegende Erfindung ein computerimplementiertes Verfahren zur zentralen Überwachung und Steuerung eines jeweiligen Batterieladevorgangs mehrerer batteriebetriebener medizinischer Geräte bereit, umfassend: Erfassen von Batterieparameterdaten der mehreren medizinischen Geräte;
Ermitteln, basierend auf den erfassten Batterieparameterdaten, eines Batterieladezustands der mehreren medizinischen Geräte;
Erzeugen eines ersten Ausgabesignals, welches dem ermittelten Batterieladezustand der mehreren medizinischen Geräte entspricht; und
Steuern, basierend auf dem erzeugten ersten Ausgabesignal, eines jeweiligen Batterieladevorgangs der mehreren medizinischen Geräte.

Das computerimplementiertes Verfahren des zweiten Aspekts kann vorzugsweise mit dem System des ersten Aspekts ausgeführt werden. In ähnlicher Weise können die verschiedenen Ausführungsformen des Systems mit dem computerimplementierten Verfahren des zweiten Aspekts durchgeführt werden.

Gemäß einem dritten Aspekt stellt die Erfindung ein Computerprogrammprodukt bereit, das ausführbaren Programmcode umfasst, welcher dazu eingerichtet ist, wenn er von einer Recheneinrichtung ausgeführt wird, dass Verfahren gemäß einer Ausführungsform des zweiten Aspekts der vorliegenden Erfindung durchzuführen.

Gemäß einem vierten Aspekt stellt die Erfindung ein nicht-flüchtiges, computerlesbares Datenspeichermedium bereit, das ausführbaren Programmcode umfasst, welcher dazu eingerichtet ist, wenn er von einer Recheneinrichtung ausgeführt wird, dass Verfahren gemäß einer Ausführungsform des zweiten Aspekts der vorliegenden Erfindung durchzuführen.

Das nicht-flüchtige, computerlesbare Datenspeichermedium kann jede Art von Computerspeicher, insbesondere Halbleiterspeicher, wie z.B. einen Festkörperspeicher, umfassen oder aus einem solchen bestehen. Das Datenträger kann auch eine CD, eine DVD, eine Blu-Ray-Disc, einen USB-Speicherstick oder dergleichen umfassen oder daraus bestehen.

Gemäß einem fünften Aspekt stellt die Erfindung einen Datenstrom bereit, der ausführbaren Programmcode umfasst oder zum Erzeugen von ausführbarem Programmcode konfiguriert ist, welcher dazu eingerichtet ist, wenn er von einer Recheneinrichtung ausgeführt wird, dass Verfahren gemäß einer Ausführungsform des zweiten Aspekts der vorliegenden Erfindung durchzuführen.

Weitere vorteilhafte Varianten, Optionen, Ausführungsformen und Modifikationen ergeben sich aus den folgenden Figuren, der ausführlichen Beschreibung, sowie aus den Ansprüchen. Es versteht sich jedoch, dass die detaillierte Beschreibung und die spezifischen Beispiele zwar bevorzugte Ausführungsformen der Erfindung angeben, aber nur zur Veranschaulichung angeführt werden, da verschiedene Änderungen und Modifikationen innerhalb des Umfangs der Erfindung für den Fachmann ersichtlich sind.

### Kurzbeschreibung der Figuren

Einzelne Ausführungsformen der vorliegenden Offenbarung werden anhand der folgenden Abbildungen im Detail erläutert werden. Die Bestandteile in den Zeichnungen sind nicht notwendigerweise maßstabsgetreu, sondern dienen dazu, die Grundsätze der vorliegenden Erfindung zu veranschaulichen. Teile in den verschiedenen Abbildungen, die den gleichen Elementen oder Verfahrensschritten entsprechen, wurden in den Abbildungen mit den gleichen Bezugszeichen versehen. Die Nummerierung von Verfahrensschritten dient zunächst nur zu deren Unterscheidung und impliziert nicht zwingend eine entsprechende Reihenfolge, wobei es jedoch eine Variante darstellt, die Schritte in der Reihenfolge ihrer Nummerierung durchzuführen. Mehrere Schritte können auch überlappend oder gleichzeitig durchgeführt werden. Von den Figuren zeigen:
- Fig. 1: ein schematisches Blockdiagramm zum Erläutern eines Systems gemäß einer Ausführungsform des ersten Aspekts der vorliegenden Erfindung;
- Fig. 2: ein schematisches Blockdiagramm zur Erläuterung einer weiteren Ausführungsform des ersten Aspekts der vorliegenden Erfindung;
- Fig. 3: ein schematisches Flussdiagramm zur Erläuterung eines computerimplementierten Verfahrens gemäß einer Ausführungsform des zweiten Aspekts der vorliegenden Erfindung;
- Fig. 4: ein schematisches Flussdiagramm zur Erläuterung eines möglichen Anwendungsfalls unter Verwendung des Systems und des Verfahrens gemäß einer Ausführungsform der vorliegenden Erfindung;
- Fig. 5: ein schematisches Beispiel, wie ein Laden von medizinischen Geräten für eine Operation gemäß einer Ausführungsform der vorliegenden Erfindung erfolgen könnte;
- Fig. 6: ein schematisches Blockdiagramm eines Computerprogrammprodukts gemäß einer Ausführungsform des dritten Aspekts der vorliegenden Erfindung; und
- Fig. 7: ein schematisches Blockdiagramm eines nicht-flüchtigen computerlesbaren Datenspeichermediums gemäß einer Ausführungsform des vierten Aspekts der vorliegenden Erfindung.

### Detaillierte Beschreibung der Figuren

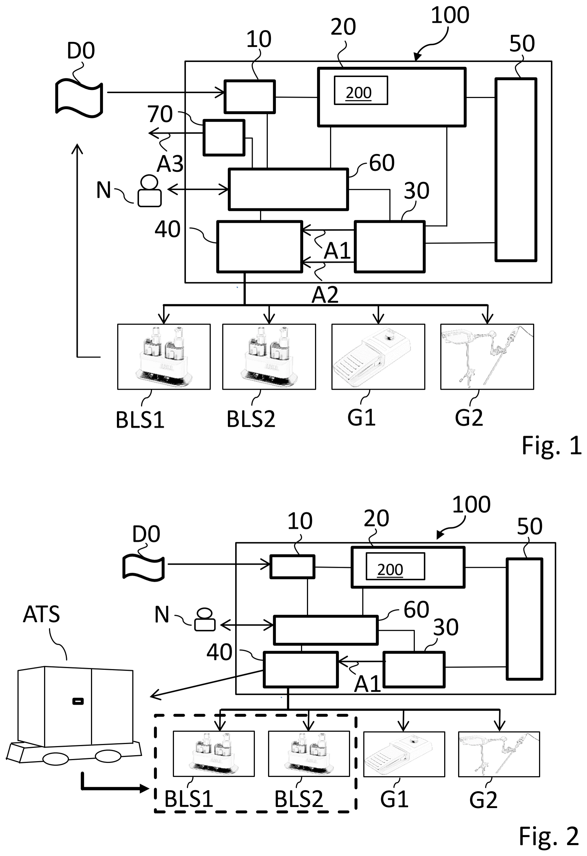

Fig. 1 zeigt ein schematisches Blockdiagramm eines Systems 100 zur zentralen Überwachung und Steuerung eines jeweiligen Batterieladevorgangs mehrerer batteriebetriebener medizinischer Geräte G1, G2 gemäß einer Ausführungsform des ersten Aspekts der vorliegenden Erfindung.

Das in der Fig. 1 dargestellte System 100 umfasst eine Eingangsschnittstelle 10, eine Recheneinrichtung 20, eine Ausgabeschnittstelle 30 und eine Steuerungseinrichtung 40.

Das System 100 gemäß der vorliegenden Erfindung kann in manchen Varianten, wie beispielsweise die Variante, die in der Fig. 1 veranschaulicht wird, auch eine Energieverwaltungseinrichtung 50, eine Benutzerschnittstelle 60 und eine Meldungseinrichtung 70 umfassen.

Die Eingangsschnittstelle 10 ist dazu eingerichtet, Batterieparameterdaten D0 einer Mehrzahl von batteriebetriebenen medizinischen Geräten G1, G2 zu erfassen. In Fig. 1 sind beispielsweise ein Fußschalter G1 und ein Haltearm eines Endoskops G2 dargestellt.

Die erfassten Batterieparameterdaten D0 können unmittelbar erhalten werden, beispielsweise wenn die Eingangsschnittstelle 10 Sensoren umfasst oder an Sensoren verbunden ist, welche verschiedene Batterieparameter der medizinischen Geräte G1, G2 messen. Die Sensoren können an den medizinischen Geräten G1, G2 angebracht sein oder werden, um das Laden/Entladen der medizinischen Geräte G1, G2 zu überwachen, und/oder an Ladestationen BLS1, BLS2 implementiert werden, um das Laden von verschiedenen Batterien zu überwachen.

Alternativ oder zusätzlich können die Batterieparameterdaten D0 aus einem PACS oder aus einer anderen Art von Datenbank stammen und aus dieser über die Eingangsschnittstelle 10 abgerufen werden.

Die Batterieparameter können thermische und/oder elektrische physikalische Größe umfassen, wie etwa die Temperatur, die Spannung oder den Strom an verschiedenen Orten der medizinischen Geräte G1, G2. Alternativ oder zusätzlich können Informationen, die vom Hersteller stammen, wie etwa die Chemie der Batterie oder die Kapazität der Batterie, Batterieparameter sein. Auch das Alter der Batterie oder Informationen über die historischen Ladungs- und Entladungszyklen der Batterie sind als Batterieparameter anzusehen.

Die Batterieparameterdaten D0 können als Bilddaten, Textdaten oder als andere Signale von den Sensoren übermittelt und über die Eingangsschnittstelle 10 erfasst werden. Die Eingangsschnittstelle 10 kann dazu eingerichtet sein, die Batterieparameterdaten D0 direkt von mindestens einer Messungseinheit, einer Bildgebungseinrichtung, oder durch eine Benutzerschnittstelle zu erhalten.

Das System 100 umfasst außerdem eine Recheneinrichtung 20, welche dazu eingerichtet ist, auf Basis der erfassten Batterieparameterdaten D0 mindestens einen, oder einen jeweiligen, Batterieladezustand der mehreren medizinischen Geräte G1, G2 zu ermitteln. Die Recheneinrichtung 20 kann als jede Vorrichtung oder jedes Mittel zum Rechnen realisiert werden, insbesondere zum Ausführen einer Software, einer App oder eines Algorithmus.

Vorteilhaft umfasst die Recheneinrichtung 20 ein künstliche-Intelligenz-Modul 200, welche eine künstliche-Intelligenz-Entität, KIE, implementiert, die dafür trainiert und eingerichtet ist, eine Lade- und/oder Entladestrategie der medizinischen Geräte G1, G2 zu erstellen.

Diese Strategien können unter Verwendung eines Modells der Batterien oder einer Simulation der Batterien (insbesondere eines digitalen Zwillings der Batterien) erstellt werden. Außerdem kann die von der KIE ermittelte Lade- und/oder Entladestrategie der verschiedenen medizinischen Geräte G1, G2 zumindest auf Informationen über einer OP-Planung basieren. Die OP-Planung kann beispielsweise von der Eingangsschnittstelle 10 empfangen werden. Die KIE kann beispielsweise den Entladezeitpunkt einer Batterie vorhersagen und diesen Zeitpunkt mit einer vorhersagten Belegung der Batterieladestationen vergleichen, so dass das Laden der Batterie im Netz der Batterieladestationen BLS1, BLS2 gewährleistet werden kann.

Die KIE kann ferner dazu ausgelegt sein, Abweichungen der OP-Planung zu erkennen und darauf basierend einen Batterieladezustand der mehreren medizinischen Geräte G1, G2, G3 am Ende der Operation (OP) entsprechend der OP-Planung vorherzusagen. In anderen Worten kann die KIE beurteilen, unter Berücksichtigung von Abweichungen der OP-Planung, die in Echtzeit erkannt werden können, ob der Entladezeitpunkt eines oder mehrerer medizinischer Geräte G1, G2, G3 vor oder nach dem Ende einer OP liegt. Die KIE kann aus (oder: basierend auf) den Bilddaten eines Endoskops oder einer Raumkamera bzw. aus den Gerätedaten und den erkannten Phasen einer OP Abweichungen von der OP-Planung erkennen. Darauf basierend kann die KIE ermitteln, ob der jeweils prognostizierte Batterieladezustand der verschiedenen in einer OP eingesetzten medizinischen Geräte (G1, G2, G3) bis zum Ende der OP ausreicht bzw. ob ein jeweils vordefinierter Mindestbatterieladezustand der medizinischen Geräte (G1, G2, G3) bis zum Ende der OP erreicht (und/oder gegebenenfalls unterschritten) wird.

Die Ausgabeschnittstelle 30 ist dazu eingerichtet, verschiedene Ausgabesignale A1, A2 zu erzeugen. Das erste Ausgabesignal A1 entspricht dem ermittelten Batterieladezustand der mehreren medizinischen Geräte G1, G2 und basiert auf der Ermittlung der Recheneinrichtung 20, vorzugsweise durch das künstliche-Intelligenz-Modul 200.

Das zweite Ausgabesignal A2 signalisiert einen erforderlichen Batteriewechsel und/oder Gerätewechsel (oder auch einen Akkuwechsel). Stellt die Recheneinrichtung 20 fest, dass der Batterieladezustand eines Gerätes G1, G2 unter einem bestimmten Sollwert liegt, kann ein Nutzer N durch das Ausgabesignal A2 über den erforderlichen Wechsel informiert werden.

Die Steuerungseinrichtung 40 ist dazu eingerichtet, basierend auf dem erzeugten ersten Ausgabesignal A1 einen jeweiligen Batterieladevorgang der mehreren medizinischen Geräte G1, G2 zu steuern. Die Steuerungseinrichtung 40 kann mit den medizinischen Geräten G1, G2 verbunden sein, vorzugsweise kabellos. Die Steuerungseinrichtung 40 ist ferner mit mehreren Batterieladestationen BLS1, BLS2 verbindbar, um den jeweiligen Batterieladevorgang von den Batterien der mehreren medizinischen Geräte G1, G2 zu steuern, insbesondere wenn die Geräte G1, G2 keinen Akku besitzen und die Batterien nicht auf dem Gerät geladen werden können. Die Steuerungseinrichtung 40 kann mit einer Anzahl von Batterieladestationen BLS1, BLS2 vernetzt werden, um die passende Batterieladestation BLS1, BLS3 für das Laden einer Batterie zu finden.

Die Steuerungseinrichtung 40 kann ferner dazu eingerichtet werden, eine Notabschaltung eines Batterieladevorgangs durchzuführen, falls die zugehörigen Batterieparameterdaten D0 außerhalb bestimmter Werten oder Wertebereiche liegen. Dadurch kann kritischen Situationen, wie etwa einer Überhitzung oder einem Kurzschluss bei einem Laden, vorgebeugt werden, um Schäden an den medizinischen Geräten G1, G2 zu verhindern.

Die dargestellte Ausführungsform in Fig. 1 umfasst ferner eine Energieverwaltungseinrichtung 50, welche dazu eingerichtet ist, einen Energieverbrauch der mehreren medizinischen Geräte G1, G2 zu optimieren. Die Energieverwaltungseinrichtung 50 kann ferner ein fehlerhaftes oder nicht optimales Laden und/oder Entladen einer Batterie erkennen und an die Steuerungseinrichtung 40 entsprechende Anweisungen übertragen. Beispielsweise kann die Energieverwaltungseinrichtung 50 eine automatische Abschaltung eines medizinischen Gerätes G1, G2 veranlassen oder einen Standby-Modus für ein medizinisches Gerät G1, G2 empfehlen, z.B. bei Nicht-Nutzung, um einen Energieverbrauch der Batterie zu optimieren. Die Energieverwaltungseinrichtung 50 kann ferner mit der Ausgabeschnittstelle 40 verbunden sein, um Vorwarnungen an einen Nutzer N zu übermitteln. In einigen Ausführungsformen kann die Energieverwaltungseinrichtung 50 in der Recheneinrichtung 20 implementiert sein.

Fig. 1 enthält ferner eine Benutzerschnittstelle 60, die dazu eingerichtet ist, Eingaben von einem Nutzer N zu empfangen und Informationen über die Überwachung und Steuerung der medizinischen Geräte G1, G2 an eine Anzeigeeinrichtung zu übermitteln. Die Benutzerschnittstelle 60 ermöglicht es einem Nutzer N auf einfache Weise, Informationen an das System der Erfindung übermitteln zu können. Die Benutzerschnittstelle 60 kann vorzugsweise eine graphische Schnittstelle umfassen und als Tablet realisiert werden. Die Benutzerschnittstelle 60 kann außerdem mit einem Tablet oder einem Mobiltelefon verbunden sein, so dass der Nutzer N mit seinen eigenen Geräten mit dem System 100 kommunizieren kann. Eine solche Benutzerschnittstelle 60 kann beispielsweise als graphische Benutzerschnittstelle durch eine Anzeigeeinrichtung des Systems 100 implementiert sein, insbesondere durch einen Bildschirm oder einen Touchscreen.

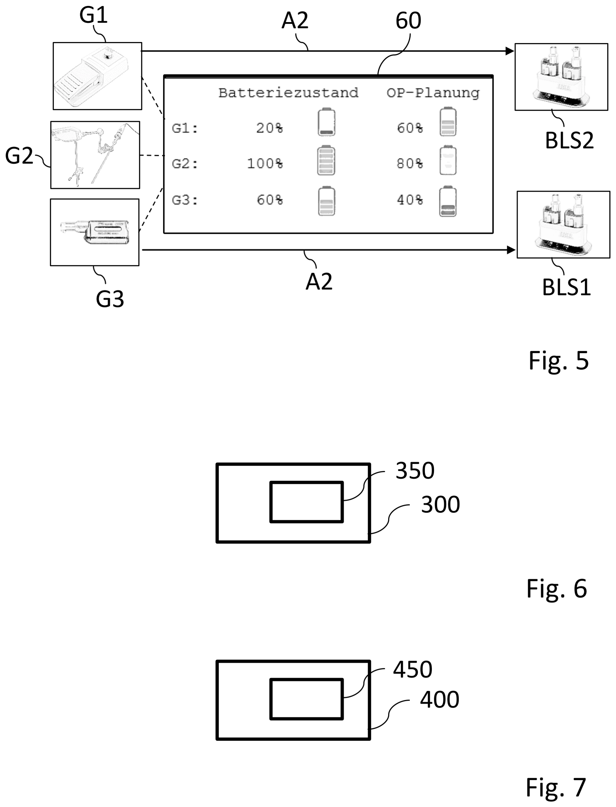

Die Benutzerschnittstelle 60 kann mit der Eingangsschnittstelle 10 verbunden sein, um Informationen über OP-Planungen oder Benutzerpräferenzen im Zusammenhang mit einer Prozedur zu erhalten. Die Benutzerschnittstelle 60 kann ebenfalls die Ausgabesignale A1, A2 der Ausgabeschnittstelle 30 oder eine darauf basierende Anzeige darstellen, beispielweise den Batterieladezustand eines medizinischen Gerätes G1, G2 (siehe die Beschreibung im Zusammenhang mit der Fig. 5), die ermittelte Ladeplanung der Recheneinrichtung 20, den geplanten Einsatz eines Gerätes G1, G2 gemäß einer OP-Planung, oder den Ladezustand der Batterien an den Ladestationen BLS1, BLS2.

Das System der Fig. 1 zeigt ferner eine optionale Meldungseinrichtung 70, welche dazu eingerichtet ist, eine Anzahl der mehreren medizinischen Geräte G1, G2 auf der Grundlage mindestens einer OP-Planung auszuwählen und für jedes der ausgewählten medizinischen Geräte G1, G2 einen entsprechenden OP-Saal zuzuweisen. Dadurch können bestimmte medizinische Geräte G1, G2 identifiziert und einer Operation (und einem zugehörigen Operationssaal) zugeordnet werden.

In manchen Ausführungsformen kann die Auswahl der medizinischen Geräte G1, G2 für eine spezifische Operation mindestens teilweise von einem Nutzer N ausgewählt werden. In diesen Ausführungsformen ist die Meldungseinrichtung 70 vorzugsweise mit der Benutzerschnittstelle 60 verbunden und dazu eingerichtet, Bildgebungsdaten oder Texteingaben zu empfangen. In einer bevorzugten Ausführungsform der Erfindung ist die Meldungseinrichtung 70 mit einem Tablet oder Mobiltelefon verbunden oder verbindbar, so dass durch Einscannen eines Barcodes oder eines QR-Codes von dem Nutzer N ausgewählte medizinische Geräte G1, G2 automatisch durch das System 100 identifiziert werden können. In diesen Ausführungsformen kann die Meldungseinrichtung 70 eine Bilderkennungseinheit umfassen, die eine App ausführen kann, um eine OP-Planung und die gewünschten medizinischen Geräte G1, G2 zu empfangen und eine Darstellung des von der Recheneinrichtung 20 ermittelten Batterieladezustands der medizinischen Geräte G1, G2, deren Verfügbarkeit und die Ladeplanung gemäß der OP-Planung zu erzeugen.

Die Meldungseinrichtung 70 kann zusätzlich eine Sicherheitsfunktion ausführen. Die Anmeldung der medizinischen Geräte G1, G2 kann mit einem Trackingsystem gekoppelt werden. Dies kann über GPS-Chips erfolgen, die an den medizinischen Geräten G1, G2 angebracht sind. Vorzugsweise können U1 (Ultra-Breitband) Chips verwendet werden, so dass das Tracking nicht notwendigerweise von Bluetooth und Wi-Fi Konnektivität abhängig sein muss.

Das Tracking kann auch mittels RFID-Technologie (Radio-Frequency Identification) erfolgen. Die Meldungseinrichtung 70 kann ein Warnsignal A3 ausgeben, wenn sich ein medizinisches Gerät G1, G2 für eine OP-Planung nicht im vorgesehenen OP-Saal befindet oder aus dem OP-Saal entfernt wurde (insbesondere ohne Abmeldung). Das Warnsignal A3 kann in der Benutzerschnittstelle 60 oder auf einem Benutzergerät (Tablet oder Mobiltelefon) angezeigt werden. Das Warnsignal A3 kann ein akustisches Signal auslösen.

Wie im Vorangehenden bereits erklärt wurde, müssen die Module, Einrichtungen und Schnittstellen des Systems 100 nicht zwangsläufig als klar unterscheidbare Einheiten implementiert sein. Manche der Module, Einrichtungen und Schnittstellen können daher in ein und demselben Gerät oder in ein und derselben Vorrichtung implementiert sein.

Fig. 2 zeigt ein schematisches Blockdiagramm zur Erläuterung einer weiteren Ausführungsform des Systems 100 des ersten Aspekts der vorliegenden Erfindung. In dieser Variante ist die Steuerungseinrichtung 40 mit einem autonomen Transportsystem ATS verbunden. Das Transportsystem ATS kann eine Mehrzahl (eine Flotte) von Transportfahrzeugen umfassen, die dazu ausgelegt sind, die zu ladenden Batterien abzuholen und sie an die von der Steuerungseinrichtung 40 ausgewählten Ladestationen BLS1, BLS2 zu bringen. In diesen Ausführungsformen der Erfindung ist das Laden von Batterien somit vorteilhaft voll automatisiert, d.h. die Batterien brauchen nicht vom Krankenhauspersonal an die Ladestationen BLS1, BLS2 gebracht zu werden, was zu einer erhöhten Effizienz und geringerer Fehlerquote führt. Die Steuerungseinrichtung 40 steuert das autonome Transportsystem ATS auf Basis des zweiten Ausgabesignals A2 der Ausgangsschnittstelle 30, welches einen Batteriewechsel und/oder einen Gerätewechsel signalisiert.

Nach dem Laden der medizinischen Geräte G1, G2 und in Abhängigkeit von der OP-Planung können die Transportfahrzeuge die aufgeladenen medizinischen Geräte G1, G2 gemäß einer oder mehrerer OP-Planungen auf die entsprechenden Operationssäle einer medizinischen Einrichtung verteilen.

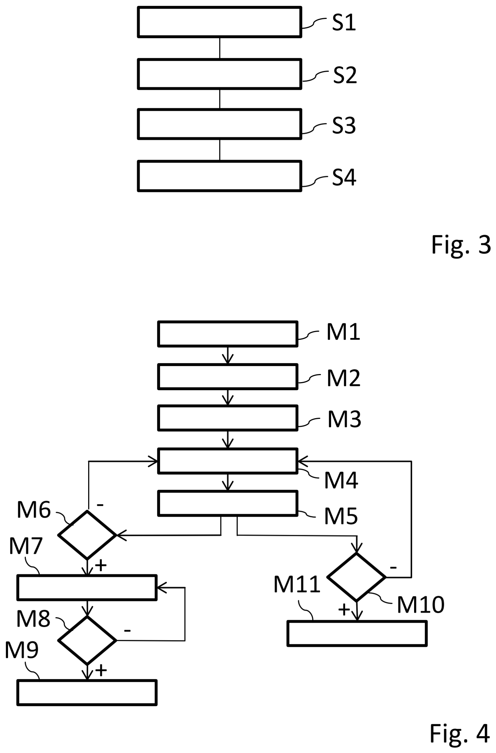

Fig. 3 zeigt ein schematisches Flussdiagramm zur Erläuterung eines computerimplementierten Verfahrens zur zentralen Überwachung und Steuerung des jeweiligen Batterieladevorgangs mehrerer batteriebetriebener medizinischer Geräte gemäß einer Ausführungsform des zweiten Aspekts der vorliegenden Erfindung.

Das Verfahren gemäß Fig. 3 ist insbesondere mittels des Systems 100 von Fig. 1 durchführbar und kann daher gemäß allen im Zusammenhang mit dem erfindungsgemäßen System 100 beschriebenen Optionen oder Varianten angepasst werden und umgekehrt. Um die Erläuterung zu verdeutlichen werden die verschiedenen Begriffe, die im Zusammenhang mit der Beschreibung von Fig. 3 verwendet werden, auf die Bezugszeichen der Fig. 1 bezogen. Dies sollte aber nicht als eine Einschränkung des Verfahrens angesehen werden.

In einem Schritt S1 werden Batterieparameterdaten D0 von mindestens einem Batterieparameter der mehreren medizinischen Geräte G1, G2 erfasst. Diese Batterieparameterdaten D0, wie im Vorangehenden bereits erklärt wurde, können von Sensoren übermittelt oder aus einer Datenbank abgerufen werden. Die Batterieparameterdaten D0 können anhand der Eingangsschnittstelle 10 erfasst oder erhalten werden.

In einem Schritt S2 wird, auf Basis der erfassten Batterieparameterdaten D0, ein (jeweiliger) Batterieladezustand der mehreren medizinischen Geräte G1, G2 ermittelt. Dies kann mit Hilfe einer Recheneinrichtung 20 erfolgen, insbesondere einer Recheneinrichtung 20, die eine künstliche-Intelligenz-Entität KIE aufweist (oder: implementiert).

In einem Schritt S3 wird ein erstes Ausgabesignal A1, welches dem ermittelten Batteriezustand der mehreren medizinischen Geräte G1, G2 entspricht (oder: diesen anzeigt), erzeugt. Dieses erste Ausgabesignal A1 kann von der Ausgabeschnittstelle 30 der Fig. 1 oder Fig. 2 generiert werden.

In einem Schritt S4 wird, basierend auf dem erzeugten ersten Ausgabesignal A1, der jeweilige Batterieladevorgang der mehreren medizinischen Geräte G1, G2 gesteuert. Die Steuerung kann von einer Steuerungseinrichtung 40 durchgeführt werden, welche mit den medizinischen Geräten G1, G2 und/oder einer Mehrzahl von Ladestationen BLS1, BLS2 verbunden ist. In manchen Ausführungsformen kann das Steuern mit Hilfe eines autonomen Transportsystems ATS (z.B. einer Flotte von autonomen Transportfahrzeuge), wie in der Fig. 2 dargestellt, erfolgen.

Weitere Optionen wurden im Vorangehenden bereits beispielhaft anhand der Beschreibung der Fig. 1 und Fig. 2 erläutert.

Fig. 4 zeigt ein schematisches Flussdiagramm zur Erläuterung eines möglichen Anwendungsfalls unter Verwendung des Systems 100 und des Verfahrens gemäß einer Ausführungsform der vorliegenden Erfindung.

In einem Schritt M1 wird zunächst eine bestimmte Anzahl von medizinischen Geräten G1, G2, Instrumenten oder Zubehör im Zusammenhang mit einer Operation identifiziert. Dies kann durch eine OP-Planung an die Eingangsschnittstelle 10 übermittelt werden, oder die relevanten medizinischen Geräte G1, G2 können von einem Nutzer N z.B. mit einem Tablet oder Mobiltelefon gescannt werden. Das Scannen kann durch eine Bildgebungseinrichtung oder durch ein Identifikationsmerkmal (Barcode oder QR-Code) erfolgen. Alle Information können von der Meldungseinrichtung 70 erfasst werden.

In einem Schritt M2 werden die medizinischen Geräte G1, G2 entsprechend einer OP-Planung angeordnet. Die OP-Planung kann mindestens Informationen über die benötigten medizinischen Geräte G1, G2 und die Art der Operation (Startzeit und voraussichtliche Dauer der Operation) enthalten.

In einem Schritt M3 wird das Tracking der identifizierten medizinischen Geräte G1, G2 initialisiert und die medizinischen Geräte G1, G2 werden einem OP-Saal zugeordnet. Das Tracking kann mit verschiedenen Technologien erfolgen, entweder mit montierten GPS Chips, Ultrabreitband-Chips oder anderen Methoden der RFID-Technologie.

In einem Schritt M4 wird eine Überwachung des jeweiligen Batterieladevorgangs der verschiedenen Geräte G1, G2 durchgeführt. Dies umfasst das Empfangen oder Erfassen von Batterieparameterdaten D0 von einem oder mehreren Batterieparametern (wie etwa Temperatur, Spannung, Strom oder Energieverbrauch) der verschiedenen medizinischen Geräte G1, G2. Basierend auf den erfassten Batterieparameterdaten D0 wird in einem Schritt M5 der Batterieladezustand der identifizierten medizinischen Geräte G1, G2 ermittelt. Dies kann in einer Recheneinrichtung 20 erfolgen, welche Algorithmen für eine solche Ermittlung aufweist. Informationen über den Batterieladezustand können auf einer Benutzerschnittstelle 60 angezeigt werden, so dass die Informationen einem Nutzer N in Echtzeit zur Verfügung stehen. Die Batterieparameterdaten D0 der Batterieparameter können ebenfalls übermittelt werden.

In einem Schritt M6 wird festgestellt, basierend auf Informationen bezüglich der OP-Planung und anhand von Modellen oder Simulationen der Batterien, ob der Batterieladezustand der medizinischen Geräte G1, G2 für die geplante Operation zu niedrig ist. Ist dies der Fall (in Fig. 4 mit "+" bezeichnet) wird über die Ausgabeschnittstelle 40 ein erstes Ausgabesignal A1 erzeugt und dementsprechend das Laden (Schritt M7) der betroffenen medizinischen Geräte G1, G2 veranlasst.

Die Steuerung des Ladens wird durch eine Steuerungseinrichtung 40 durchgeführt, die mit den medizinischen Geräten G1, G2, einer Mehrzahl von Ladestationen BLS1, BLS2 und/oder einer Flotte von autonomen Transportfahrzeugen verbunden oder verbindbar ist. Stellt die Recheneinrichtung 20 fest, dass der Batterieladezustand eines medizinischen Gerätes G1, G2 für die relevante OP-Planung in Ordnung ist (in Fig. 4 mit "-" bezeichnet), so wird kein Laden veranlasst und das System 100 fährt mit der Überwachung (Schritt M4) der verschiedenen Batterien der medizinischen Geräte G1, G2 fort. In diesem Fall ist es auch möglich, dass manche der medizinischen Geräte G1, G2 (durch die Steuerungseinrichtung 40) in einen Standby-Modus geschaltet werden, um Energie zu sparen.

Während des Ladens einer Batterie in Schritt M7 kontrolliert die Steuerungseinrichtung 40 in einem Schritt M8, dass bestimmte Parameter (Temperatur, Spannung, Strom usw.) innerhalb bestimmter Werte oder Wertebereiche liegen, um Schäden an den medizinischen Geräten G1, G2 zu vermeiden. Wenn bestimmte Werte auf eine Überhitzung oder einen Kurzschluss hindeuten (in Fig. 4 mit "+" bezeichnet), führt die Steuerungseinrichtung 40 in einem Schritt M9 eine Notabschaltung des zugehörigen Batterieladevorgangs durch. Ansonsten (in Fig. 4 mit "-" bezeichnet) wird das Laden wie geplant durchgeführt.

Nach der Anmeldung des Trackingsystems eines medizinischen Gerätes G1, G2 wird in einem Schritt M10 ständig geprüft, ob das medizinische Gerät G1, G2 ohne Abmeldung aus dem gemäß OP-Planung entsprechenden OP-Saal entfernt wird. In einer solchen Situation (in Fig. 4 mit "+" bezeichnet) kann das System 100 über die Meldungseinrichtung 70 ein Warnsignal A3 ausgeben (Schritt M11). Zusätzlich kann beispielsweise ein akustisches Signal ausgelöst werden.

Die Schritte M6 bis und M11 können auch während der Durchführung einer geplanten Operation aktiv sein. Beispielsweise wird das Tracking der medizinischen Geräte G1, G2 vorzugsweise erst am Ende der Operation deaktiviert.

In Fig. 5 ist ein schematisches Beispiel dargestellt, welches beschreibt, wie ein Laden von medizinischen Geräten G1, G2, G3 für eine Operation gemäß einer Ausführungsform der vorliegenden Erfindung beispielhaft erfolgen kann.

Fig. 5 zeigt eine Mehrzahl von batteriebetriebenen medizinischen Geräten G1 bis G3. In Fig. 5 sind ein Fußschalter G1, ein Haltearm eines Endoskop G2 und eine LED-Lichtquelle G3 dargestellt. Diese medizinischen Geräte G1-G3 können gemäß einer OP-Planung für eine bestimmte Operation verwendet werden. Die medizinischen Geräte G1 bis G3 der Fig. 5 können beispielsweise von der Meldungseinrichtung 70 ausgewählt werden und einer OP-Saal zugeordnet werden.

Die Batterieparameterdaten D0 werden zunächst von der Eingangsschnittstelle 10 des erfindungsgemäßen Systems 100 erfasst, und auf dieser Basis ermittelt die Recheneinrichtung 20 den jeweiligen Batterieladezustand. Ein mögliches Ergebnis ist auf der linken Spalte der Benutzerschnittstelle 60 der Fig. 5 angezeigt.

Zusätzlich werden Informationen bezüglich der OP-Planung der Operation an das System 100 geliefert (rechte Spalte der Benutzerschnittstelle 60), anhand derer die Recheneinrichtung 20 den voraussichtlich erforderlichen Batterieladezustand der verschiedenen medizinischen Geräte G1-G3 ermittelt. Die OP-Planung liefert beispielsweise den jeweiligen Mindest-Ladezustand der medizinischen Geräte G1 bis G3 für eine Operation. Die auf der rechten Spalte der Benutzerschnittstelle 60 dargestellten Informationen können auch benutzerspezifische Einstellungen entsprechen. Beispielsweise können zwei Nutzer die gleiche Prozedur auf unterschiedliche Weise durchführen, was dazu führt, dass der voraussichtliche Energieverbrauch der medizinischen Geräte G1 bis G3 vom Nutzer abhängt. Dies kann bei der Ermittlung der Recheneinrichtung 20 berücksichtigt werden.

Der Fußschalter G1 weist eine Batterie auf, welche zu 20% geladen ist. Für die geplante Operation sind jedoch mindestens 60% erforderlich. Daher wird ein erstes Ausgabesignal A1 von der Ausgabeschnittstelle 30 erzeugt. Dementsprechend wird die Steuerungseinrichtung 40 das Laden des Fußschalters G1 veranlassen. Die Ausgabeschnittstelle 30 gibt dementsprechend ein zweites Ausgabesignal A2 aus. Unter Berücksichtigung der Belegung der Ladestationen BLS1, BLS2 und der Startzeit der Operation gemäß OP-Planung wird der Fußschalter G1 in der Ladestation BLS2 auf mindestens 60% geladen.

Der Haltearm G2 eines Endoskops ist voll aufgeladen. Für die Operation muss die Batterie mindestens zu 80% geladen sein. Diese Voraussetzung ist somit bereits erfüllt, und kein Laden ist erforderlich. Das erfindungsgemäße System 100, insbesondere die Steuerungseinrichtung 40, kann sicherstellen, dass die Batterie des Haltearms G2 bis zur Startzeit der Operation nicht unter 80% entladen wird. Eine beispielsweise durchführbare Maßnahme wäre es, den Haltearm G2 von der Steuerungseinrichtung 40 in einen Standby-Modus zu versetzen.

Die LED-Lichtquelle G3 ist zu 60% geladen. Für die geplante Operation sind nur 40% erforderlich. Die Recheneinrichtung 20 stellt fest, basierend auf der Startzeit der Operation, dass ein Laden erforderlich ist. Der Grund dafür könnte sein, dass die LED-Lichtquelle G3 zu diesem Zeitpunkt in einer anderen Operation wird. Die Recheneinrichtung 20 prognostiziert einen Batterieladezustand der LED-Lichtquelle G3 unter 40% am Ende der aktuellen Operation. Daher ist ein Ladevorgang erforderlich, um die LED-Lichtquelle G3 für die zweite Operation einsatzbereit zu machen. Die Ausgabeschnittstelle 30 gibt dementsprechend ein zweites Ausgabesignal A2 aus. Unter Berücksichtigung der Belegung der Ladestationen BLS1, BLS2 und der Startzeit der Operation gemäß OP-Planung wird die LED-Lichtquelle G3 in der Ladestation BLS1 auf mindestens 40% geladen.

Fig. 6 zeigt ein schematisches Blockdiagramm eines Computerprogrammprodukts 300 gemäß einer Ausführungsform des dritten Aspekts der vorliegenden Erfindung. Das Computerprogrammprodukt 300 umfasst ausführbaren Programmcode 350, welcher dazu eingerichtet ist, wenn er ausgeführt wird (z.B. durch eine Recheneinrichtung), das Verfahren gemäß einer Ausführungsform der vorliegenden Erfindung durchzuführen, beispielsweise gemäß Fig. 3 oder Fig. 4.

Fig. 7 zeigt ein schematisches Blockdiagramm eines nicht-flüchtigen computerlesbaren Datenspeichermediums 400 gemäß einer Ausführungsform der vorliegenden Erfindung. Das Datenspeichermedium 400 umfasst ausführbaren Programmcode 450, welcher dazu eingerichtet ist, wenn er ausgeführt wird (z.B. durch eine Recheneinrichtung), das Verfahren gemäß einer Ausführungsform der vorliegenden Erfindung durchzuführen, beispielsweise gemäß Fig. 3 oder Fig. 4.

Das nicht-flüchtige computerlesbare Datenspeichermedium 400 kann beispielsweise als ein Halbleiterspeicher, z.B. ein SSD-Speicherstein ausgebildet sein oder einen solchen aufweisen. Das Datenspeichermedium 400 kann auch eine CD, DVD, Blu-Ray oder eine magnetische Speichervorrichtung aufweisen oder umfassen.

Die vorstehende Beschreibung der offenbarten Ausführungsformen enthält lediglich Beispiele für mögliche Umsetzungen, die beschrieben werden, um einen Fachmann in die Lage zu versetzen, die vorliegende Erfindung herzustellen oder verwenden zu können. Verschiedene Variationen und Modifikationen dieser Ausführungsformen sind für den Fachmann - nach Kenntnis der vorliegenden Erfindung - leicht ersichtlich, und die hierin definierten allgemeinen Prinzipien können auf andere Ausführungsformen angewendet werden, ohne vom Umfang der vorliegenden Offenbarung abzuweichen.

Somit soll die vorliegende Erfindung nicht auf die hierin gezeigten, spezifischen Ausführungsformen beschränkt sein, sondern ihr soll der breiteste Umfang zugestanden werden, der mit den hierin offenbarten Prinzipien und Merkmalen übereinstimmt.

### Bezugszeichenliste

- 10: Eingangsschnittstelle
- 20: Recheneinrichtung
- 30: Ausgabeschnittstelle
- 40: Steuerungseinrichtung
- 50: Energieverwaltungseinrichtung
- 60: Benutzerschnittstelle
- 70: Meldungseinrichtung
- 100: System
- 200: Künstliche-Intelligenz-Modul
- 300: Computerprogrammprodukt
- 350: Programmcode
- 400: Datenspeichermedium
- 450: Programmcode
- A1, A2: Ausgabesignale
- A3: Warnsignal
- ATS: Autonomes Transportsystem
- BLS1, BLS2: Ladestationen
- D0: Batterieparameterdaten
- N: Nutzer
- G1: Fußschalter
- G2: Haltearm eines Endoskops
- G3: LED-Lichtquelle
- S1..S4; M1..M11: Verfahrensschritte

## Patentansprüche

1. System (100) zur zentralen Überwachung und Steuerung eines jeweiligen Batterieladevorgangs mehrerer batteriebetriebener medizinischer Geräte (G1, G2, G3), umfassend:
eine Eingangsschnittstelle (10), welche dazu eingerichtet ist, Batterieparameterdaten (D0) der mehreren medizinischen Geräte (G1, G2, G3) zu erfassen;
eine Recheneinrichtung (20), welche dazu eingerichtet ist, basierend auf den erfassten Batterieparameterdaten (D0) einen jeweiligen Batterieladezustand der mehreren medizinischen Geräte (G1, G2, G3) zu ermitteln;
eine Ausgabeschnittstelle (30), welche dazu eingerichtet ist, ein erstes Ausgabesignal (A1) zu erzeugen, welches mindestens einem der ermittelten Batterieladezustand der mehreren medizinischen Geräte (G1, G2, G3) entspricht; und
eine Steuerungseinrichtung (40), welche dazu eingerichtet ist, basierend auf dem erzeugten ersten Ausgabesignal (A1) einen jeweiligen Batterieladevorgang der mehreren medizinischen Geräte (G1, G2, G3) zu steuern.

2. System (100) nach Anspruch 1,
wobei die Eingangsschnittstelle (10) Sensoren umfasst, die dazu ausgelegt sind, elektrische und/oder thermische Batterieparameterdaten (D0) zu erfassen.

3. System (100) nach Anspruch 1 oder Anspruch 2,
wobei die Steuerungseinrichtung (40) mit mehreren Batterieladestationen (BLS1, BLS2) verbindbar ist, um den jeweiligen Batterieladevorgang der mehreren medizinischen Geräte (G1, G2, G3) zu steuern.

4. System (100) nach einem der Ansprüche 1 bis 3, ferner umfassend eine Energieverwaltungseinrichtung (50), welche dazu eingerichtet ist, einen Energieverbrauch der mehreren medizinischen Geräte (G1, G2, G3) zu optimieren.

5. System (100) nach einem der Ansprüche 1 bis 4,
wobei die Recheneinrichtung (20) ein künstliches-Intelligenz-Modul (200) umfasst, welches eine künstliche-Intelligenz-Entität, KIE, implementiert, die dafür trainiert und eingerichtet ist, eine Lade- und/oder Entladestrategie der mehreren medizinischen Geräte (G1, G2, G3) zu erstellen.

6. System (100) nach Anspruch 5,
wobei die Lade- und/oder Entladestrategie der mehreren medizinischen Geräte (G1, G2, G3) zumindest auf Informationen bezüglich einer OP-Planung basiert.

7. System (100) nach Anspruch 6,
wobei die KIE ferner dafür trainiert und eingerichtet ist, Abweichungen der OP-Planung zu erkennen und darauf basierend einen Batterieladezustand der mehreren medizinischen Geräte (G1, G2, G3) am Ende der OP entsprechend der OP-Planung vorherzusagen.

8. System (100) nach einem der Ansprüche 1 bis 7,
ferner umfassend eine Benutzerschnittstelle (60), die dazu eingerichtet ist, Eingaben von einem Nutzer (N) zu empfangen und Informationen über die Überwachung und Steuerung der medizinischen Geräte (G1, G2, G3) an eine Anzeigeeinrichtung zu übermitteln.

9. System (100) nach einem der Ansprüche 1 bis 8,
ferner umfassend eine Meldungseinrichtung (70), welche dazu eingerichtet ist, eine Anzahl der mehreren medizinischen Geräte (G1, G2, G3) auf der Grundlage mindestens einer OP-Planung auszuwählen und für jedes der ausgewählten medizinischen Geräte (G1, G2, G3) einen entsprechenden OP-Saal zuzuweisen.

10. System (100) nach Anspruch 9, wobei die Meldungseinrichtung (70) ein Warnsignal (A3) ausgibt, wenn sich ein gruppiertes medizinisches Gerät (G1, G2, G3) nicht im vorgesehenen OP-Saal befindet.

11. System (100) nach einem der Ansprüche 1 bis 10, wobei die Steuerungseinrichtung (40) ferner dazu eingerichtet ist, eine Notabschaltung eines Batterieladevorgangs durchzuführen, falls die zugehörigen Batterieparameterdaten (D0) außerhalb von bestimmten Werten liegen.

12. System (100) nach einem der Ansprüche 1 bis 11, wobei die Ausgabeschnittstelle (30) ferner dazu eingerichtet ist, ein zweites Ausgabesignal (A2) zu erzeugen, welches einen Batteriewechsel und/oder einen Gerätewechsel signalisiert.

13. System (100) nach Anspruch 12, wobei die Steuerungseinrichtung (40) ferner dazu eingerichtet ist, auf Basis des zweiten Ausgabesignals (A2), welches einen Batteriewechsel und/oder einen Gerätewechsel signalisiert, ein autonomes Transportsystem (ATS) zu steuern.

14. Computerimplementiertes Verfahren zur zentralen Überwachung und Steuerung eines jeweiligen Batterieladevorgangs mehrerer batteriebetriebener medizinischer Geräte (G1, G2, G3), umfassend:
Erfassen (S1) von Batterieparameterdaten (D0) der mehreren medizinischen Geräte (G1, G2, G3);
Ermitteln (S2), basierend auf den erfassten Batterieparameterdaten (D0), eines Batterieladezustands der mehreren medizinischen Geräte (G1, G2, G3);
Erzeugen (S3) eines ersten Ausgabesignals (A1), welches dem ermittelten Batterieladezustand der mehreren medizinischen Geräte (G1, G2, G3) entspricht; und
Steuern (S4), basierend auf dem erzeugten ersten Ausgabesignal (A1), eines jeweiligen Batterieladevorgangs der mehreren medizinischen Geräte (G1, G2, G3).

15. Computerprogrammprodukt (300), umfassend ausführbaren Programmcode (350), welcher dazu eingerichtet ist, wenn er ausgeführt wird, das Verfahren gemäß Anspruch 14 durchzuführen.
